(19) Europäisches Patentamt
European Patent Office
Office européen des brevets

(11) EP 4 617 309 A1

(12) **EUROPEAN PATENT APPLICATION**
published in accordance with Art. 153(4) EPC

(43) Date of publication:
**17.09.2025 Bulletin 2025/38**

(21) Application number: **23907850.4**

(22) Date of filing: **22.12.2023**

(51) International Patent Classification (IPC):
***C08L 3/14*** *(2006.01)* ***C08B 30/20*** *(2006.01)*
***C08B 31/00*** *(2006.01)* ***A61L 15/22*** *(2006.01)*

(52) Cooperative Patent Classification (CPC):
**A61L 15/22; C08B 30/20; C08B 31/00; C08L 3/14**

(86) International application number:
**PCT/KR2023/021369**

(87) International publication number:
**WO 2024/136567 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2022 KR 20220183662**

(71) Applicant: **LG CHEM, LTD.**
**Seoul 07336 (KR)**

(72) Inventors:
- **LEE, Dong Hwan**
  **Daejeon 34122 (KR)**
- **YUN, Hae Sung**
  **Daejeon 34122 (KR)**
- **HAM, Kyung Rok**
  **Daejeon 34122 (KR)**
- **CHO, Beom Shin**
  **Daejeon 34122 (KR)**
- **KANG, Sun Ah**
  **Daejeon 34122 (KR)**
- **CHUNG, Da Sol**
  **Daejeon 34122 (KR)**

(74) Representative: **Plasseraud IP**
**104 Rue de Richelieu**
**CS92104**
**75080 Paris Cedex 02 (FR)**

# (54) POLYMER

(57) The present specification discloses a polymer. The polymer may be an absorbent polymer used for forming a so-called SAP. Through adjustment of materials applied to a cross-linking process for forming the SAP, and conditions of the cross-linking process, such a polymer may be formed even without using a so-called cross-linking agent or by minimizing its used amount, thereby exhibiting excellent absorption capacity. The polymer may be formed by cross-linking a biodegradable material, even without using the cross-linking agent or by minimizing the same, thereby exhibiting the maximum biodegradability of the material. The present specification also discloses a use of the polymer.

[Figure 1]

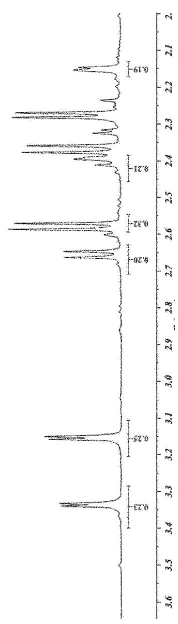

EP 4 617 309 A1

## Description

### Technical Field

This application claims the benefit of priority based on Korean Patent Application No. 10-2022-0183662 dated December 23, 2022, the disclosure of which is incorporated herein by reference in its entirety.

This specification discloses a polymer and a use thereof.

### Background Art

A hydrogel polymer or hydrogel is generally defined as a cross-linked hydrophilic polymer.

Such a polymer can be used as a material called an SAP (Super Absorbent Polymer). The SAP is a material that can absorb moisture tens to thousands of times its own weight. The SAP is used for various applications, such as sanitary products such as hygiene products or diapers, medical products, household materials, agricultural materials, horticultural materials, transportation materials, civil engineering and construction materials, materials related to electrical and electronic devices, or water treatment agents.

The most widely used hydrogel polymer, which is used as the SAP, is a polymer made of a vinyl-based material such as cross-linked polyacrylic acid.

Such materials are relatively inexpensive and have excellent absorption capacity, but cause various problems because they remain semi-permanently even after disposal.

To solve such problems, there are various attempts to manufacture the SAP from so-called biodegradable materials.

However, the materials known to date do not form the SAP with balanced physical properties. For example, the most representative physical property required for the SAP is absorption capacity, but in the SAP of a biodegradable material known to date, at least one characteristic of absorption capacity and biodegradability is not satisfactorily secured, or in some cases, both physical properties are not secured at an appropriate level.

For example, to use a biodegradable material as the SAP, the biodegradable material must be cross-linked to an appropriate level. Since the materials usually used as cross-linking agents have no or poor biodegradability, it is necessary to perform the cross-linking without using a cross-linking agent or by minimizing its used amount. However, biodegradable materials have no or poor cross-linking sites, and even when there are some cross-linking sites, there is a problem that the degree of cross-linking is lowered depending on the structure.

### Disclosure

### Technical Problem

This specification discloses a polymer. This specification is intended to disclose a content of adjusting materials applied in a cross-linking process for forming an SAP, and conditions of the cross-linking process to provide a polymer having effectively excellent absorption capacity even without using a so-called cross-linking agent or by minimizing its used amount. In addition, this specification is intended to disclose a content of forming the SAP by cross-linking a biodegradable material even without using the cross-linking agent or by minimizing the same, thereby maximizing biodegradability of the material. This specification also discloses a use of the polymer.

### Technical Solution

Among the physical properties mentioned in this specification, when the measurement temperature affects the physical property, the relevant physical property is a physical property measured at room temperature, unless otherwise specified.

In this specification, the term room temperature means a natural temperature without specifically heating or cooling, which may mean, for example, any one temperature in a range of 10°C to 30°C, or a temperature of about 23°C, about 25°C, or about 27°C or so. The unit of temperature mentioned in this specification is Celsius (°C), unless otherwise specified.

Among the physical properties mentioned in this specification, when the measurement pressure affects the physical property, the relevant physical property is a physical property measured at the normal pressure, unless otherwise specified.

In this specification, the term normal pressure means a natural pressure without special pressurization or depressurization, which may usually mean a pressure of about 730 mmHg to 790 mmHg or so.

Among the physical properties mentioned in this specification, when the measurement humidity affects the

physical property, the relevant physical property is a physical property measured at humidity which is not particularly adjusted under the room temperature and normal pressure states, unless otherwise specified.

This specification discloses a polymer.

The polymer may be a so-called cross-linked polymer, or may be a polymer before cross-linking. If the polymer is a cross-linked polymer, the polymer may be a hydrogel polymer or hydrogel.

The polymer may be an absorbent polymer.

Such an absorbent polymer may exhibit at least one of water content, centrifuge retention capacity (CRC), and absorption capacity under pressure (AUP) in the ranges described herein.

For example, the lower limit of the centrifuge retention capacity (CRC) of the polymer according to EDANA (European Disposables and Nonwovens Association) method WSP 241.3 may be 10 g/g, 15 g/g, 20 g/g, 25 g/g, 30 g/g, 35 g/g, 40 g/g, or 45 g/g or so, and the upper limit thereof may be 100 g/g, 95 g/g, 90 g/g, 85 g/g, 80 g/g, 75 g/g, 70 g/g, 65 g/g, 60 g/g, 55 g/g, 50 g/g, 45 g/g, 40 g/g, or 35 g/g or so. The centrifuge retention capacity (CRC) may be within a range of more than or equal to, or more than any one of the above-described lower limits; or within a range of less than or equal to, or less than any one of the above-described upper limits while being more than or equal to, or more than any one of the above-described lower limits. The centrifuge retention capacity may be evaluated by the method described in "1. Evaluation of centrifuge retention capacity (CRC)" of Example sections of this specification.

The lower limit of the water content of the polymer may be 40 wt%, 45 wt%, 50 wt%, or 55 wt% or so, and the upper limit thereof may be 70 wt%, 65 wt%, or 60 wt% or so. The water content may be within a range of more than or equal to, or more than any one of the above-described lower limits; or within a range of less than or equal to, or less than any one of the above-described upper limits while being more than or equal to, or more than any one of the above-described lower limits. The water content is the content of moisture contained in the polymer relative to the total weight of the polymer to be measured, which may be calculated based on the weight of the polymer containing moisture and the weight of the dried polymer. For example, the water content may be calculated through the weight loss according to evaporation of moisture in the polymer during a drying process by raising the temperature of the polymer in a crumb state through infrared heating. The drying process for measuring water content may comprise raising the temperature from room temperature to about 50°C or so, and then performing vacuum drying for about 6 hours or so while maintaining the temperature at 50°C.

The lower limit of the absorption capacity under pressure (AUP) of the polymer at 0.7 psi according to EDANA (European Disposables and Nonwovens Association) method WSP 242.3 may be 1.5 g/g, 2 g/g, 2.5 g/g, 3 g/g, 3.5 g/g, 4 g/g, 4.5 g/g, 5 g/g, or 5.5 g/g or so, and the upper limit thereof may be 40 g/g, 35 g/g, 30 g/g, 20 g/g, 15 g/g, 10 g/g, 8 g/g, 6 g/g, or 4 g/g or so. The absorption capacity under pressure (AUP) may be within a range of more than or equal to, or more than any one of the above-described lower limits; or within a range of less than or equal to, or less than any one of the above-described upper limits while being more than or equal to, or more than any one of the above-described lower limits.

If the polymer exhibits at least one characteristic of the water content, centrifuge retention capacity, and absorption capacity under pressure, it may be defined as an absorbent polymer. The polymer may exhibit any one of the water content, centrifuge retention capacity, and absorption capacity under pressure, or may exhibit two or more, or all.

The polymer may be a biodegradable polymer.

For example, the lower limit of biodegradability of the polymer may be 60%, 62%, 64%, 66%, 68%, 70%, 72%, 74%, 76%, 78%, 80%, 82%, 84%, 86%, 88%, 90%, 92%, 94%, 96%, 98%, or 99% or so, and the upper limit thereof may be 100%, 98%, 96%, 94%, 92%, 90%, 88%, 86%, 84%, 82%, 80%, 78%, or 76% or so. The biodegradability may be within a range of more than or equal to, or more than any one of the above-described lower limits; or within a range of less than or equal to, or less than any one of the above-described upper limits while being more than or equal to, or more than any one of the above-described lower limits. The polymer may exhibit the biodegradability before or after cross-linking.

As is well known, the term polymer may mean a high molecular weight material formed by linking two or more unitary bodies (e.g., monomers) by covalent bonds. The polymer may mean a material containing a structure in which two or more unitary bodies are connected by covalent bonds and simultaneously exhibiting a molecular weight of a certain level or higher. At this time, the range of the molecular weight will be described below.

The polymer may be a polysaccharide component, or may comprise the same.

The term polysaccharide component means a polysaccharide or a mixture of polysaccharides. In the case of a mixture of polysaccharides, the mixture may be a mixture of one type of polysaccharide or a mixture of two or more types of polysaccharides. Here, two or more types of polysaccharides may also mean different types of polysaccharides, and may also include a polysaccharide which is the same type of polysaccharide, but with different physical properties such as molecular weight. The polysaccharide component comprises only polysaccharides.

The term polysaccharide has a meaning known in the industry. Generally, the polysaccharide refers to polymer molecules in which two or more unitary bodies are linked by covalent bonds. The covalent bond connecting the unitary bodies is usually a glycosidic bond.

Representative polysaccharides include starch, glycogen, cellulose, chitin, or chitosan, and the like.

The unitary body forming the polysaccharide may be a biomolecule composed of carbon, hydrogen, and oxygen, or composed of carbon, hydrogen, oxygen, and nitrogen. In this specification, the term biomolecule is interpreted to have

the meaning generally applied in the industry. Typically, as an example of the biomolecule in the industry, monosaccharides such as glucose, galactose, fructose or xylose, disaccharides such as sucrose, lactose, maltose or trehalose, polyols such as sorbitol or mannitol, oligosaccharides such as maltodextrin, dextrin, raffinose, stachyose or fructo-oligosaccharide and/or amino sugars such as glucosamine or N-acetal glucosamine, and the like are known, but the types of biomolecules in the present application are not limited to the foregoing.

The lower limit of the content of the polysaccharide or polysaccharide component in the polymer may be 55 wt%, 60 wt%, 65 wt%, 70 wt%, 75 wt%, 80 wt%, 85 wt%, 90 wt%, 95 wt%, 99 wt%, or 99.5 wt% or so, and the upper limit thereof may be 100 wt%, 98 wt%, 96 wt%, 94 wt%, 92 wt%, or 90 wt% or so. The ratio may be within a range of more than or equal to, or more than any one of the above-described lower limits; or within a range of less than or equal to, or less than any one of the above-described upper limits; or within a range of less than or equal to, or less than any one of the above-described upper limits while being more than or equal to, or more than any one of the above-described lower limits.

The ratio of the polysaccharide component in the polymer is not particularly limited, but as the ratio increases, the biodegradability of the polymer increases. However, in the case of conventional absorbent materials to which the polysaccharide component is applied, if the ratio of the polysaccharide component is excessively increased in consideration of biodegradability, there is a problem that the absorption capacity is reduced. However, the polymer can stably achieve the desired absorption capacity while maintaining the ratio of the polysaccharide component at a high level.

If the polymer (e.g., the polysaccharide or polysaccharide component) is in a cross-linked state and has absorption capacity, the relevant polymer may be called the hydrogel polymer or hydrogel. In one example, the polymer may also be in a powder state formed through a grinding process or the like.

The polymer in the powder state may also be applied to additional processes such as surface cross-linking.

The polymer, or the polysaccharide or the polysaccharide component may be in a cross-linked state. The cross-linking means a state where two or more molecules of the polymer, polysaccharide, or polysaccharide component are linked by one or more chemical bonds. Typically, the cross-links are formed by compounds called so-called cross-linking agents. Such a cross-linking agent has two or more functional groups capable of reacting with the polymer, polysaccharide, or polysaccharide component, where a cross-linked structure may be formed by such functional groups.

In one example, the cross-linking of the polymer may be performed without using the cross-linking agent or while minimizing the used amount. Most of the cross-linking agents used to form SAP, which are known to date, are non-biodegradable or poorly biodegradable substances, so that even if the polysaccharide or polysaccharide component having biodegradability is used as the polymer, in most cases, its biodegradability is not effectively demonstrated after cross-linking. Therefore, if the cross-linking is performed without using a cross-linking agent or while minimizing the used amount, it is possible to obtain a polymer in which the biodegradability of the material is implemented while exhibiting the desired absorption capacity.

In this specification, the cross-linking performed without using a cross-linking agent or while minimizing the used amount as above may be referred to as self-cross-linking.

In one example, the polymer may comprise the self-cross-linked polysaccharide or self-cross-linked polysaccharide component.

For example, the lower limit of the ratio of the self-cross-linked polysaccharide or self-cross-linked polysaccharide component in the polymer based on the total weight of the polymer may be 55 wt%, 60 wt%, 65 wt%, 70 wt%, 75 wt%, 80 wt%, 85 wt%, 90 wt%, 95 wt%, 97 wt%, 99 wt%, or 99.5 wt% or so, and the upper limit thereof may be 100 wt%, 98 wt%, 96 wt%, 94 wt%, 92 wt%, or 90 wt% or so. The ratio may be within a range of more than or equal to, or more than any one of the above-described lower limits; or within a range of less than or equal to, or less than any one of the above-described upper limits while being more than or equal to, or more than any one of the above-described lower limits.

The upper limit of the ratio of the cross-linking agent in the self-cross-linked polymer, self-cross-linked polysaccharide or self-cross-linked polysaccharide component based on the total weight of the self-cross-linked polymer, self-cross-linked polysaccharide, or self-cross-linked polysaccharide component may be 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, 0.001 wt%, 0.0005 wt%, or 0.0001 wt% or so, and the lower limit thereof may be 0 wt% or so. The ratio of the cross-linking agent may be within a range of less than or equal to, or less than any one of the above-described upper limits; or within a range of less than or equal to, or less than any one of the above-described upper limits while being more than or equal to, or more than any one of the above-described lower limits. Here, the cross-linking agent is a substance to form a chemical bond linking the polymer, polysaccharide, or polysaccharide component, which means a substance other than the polymer, polysaccharide, or polysaccharide component.

In this way, when the cross-linking is performed without using a cross-linking agent or while minimizing the used amount, it is possible to utilize the biodegradability of the material itself maximally while securing the absorption capacity. Since polysaccharides or polysaccharide components, which are generally known biodegradable materials, have low cross-linking efficiencies, it is not easy to obtain a polymer having desired properties (e.g., absorption capacity). In this specification, specific types of polysaccharides or polysaccharide components capable of effectively self-cross-linking without using a cross-linking agent, and cross-linking methods are disclosed. These polysaccharides or polysaccharide

components may have, in a self-cross-linked state, excellent physical properties (e.g., absorption capacity) while having excellent biodegradation characteristics.

The self-cross-linking of the polysaccharide component may be performed using a so-called acidic polysaccharide component. The acidic polysaccharide component is a polysaccharide component having an acidic group. An example of the acidic group may include a carboxylic group, a phosphate group, a phosphite group and/or a sulfuric ester group, or salts thereof.

The acidic polysaccharide component may be adjusted for effective self-cross-linking. For example, in the polysaccharide component, F of Equation 1 below may be more than or equal to a certain level. The F is a value for the polysaccharide component before self-cross-linking.

[Equation 1]

$$F = AP \times DS \times \text{Log}(Mw)$$

In Equation 1, AP is the ratio of amylopectin in the polysaccharide component, DS is the degree of substitution of the polysaccharide component, and Mw is the molecular weight of the polysaccharide component.

In Equation 1, AP is the ratio of amylopectin in the polysaccharide component, and is measured in the manner described in "3. Content measurement of amylopectin and amylose" of the Example sections in this specification. The AP is calculated as Ap/(Am+Ap) when the sum (Am+Ap) of the weight (Am) of amylose and the weight (Ap) of amylopectin in the polysaccharide component is converted to 100. As is known, polysaccharide components often consist mainly of amylose and amylopectin. Amylose and amylopectin are composed of glucose molecules linked by glycosidic bonds, where amylose has a linear chain structure, while amylopectin has relatively short and highly branched chains. In addition, amylose crystallizes relatively easily compared to amylopectin, and amylopectin has a relatively higher solubility in water than amylose. Therefore, the ratio of amylose to amylopectin in the polysaccharide component may be related to the absorption capacity and biodegradability of the polysaccharide component. Furthermore, the ratio of amylopectin in the polysaccharide component is related to a steric structure affecting a steric hindrance of the relevant polysaccharide component, where the self-cross-linking efficiency may be affected depending on such a steric structure.

The degree of substitution DS in Equation 1 is a value indicating to what extent the hydroxy group present in each unitary body included in the polysaccharide component has been substituted with the acidic group, and is an average value for each unitary body present in the polysaccharide component. For example, if the unitary body is a grape sugar (glucose) unit, there are three hydroxy groups in the relevant unit before modification, and thus, if all the hydroxy groups are replaced with acidic groups, the degree of substitution for the relevant unit is 3. The degree of substitution of the polysaccharide component is the average value of the degree of substitution of each unitary body present in the relevant polysaccharide component, so that for example, in a polysaccharide component containing 5 grape sugar (glucose) units, if the degree of substitution of each unit is 1, 0, 2, 3, and 1, the degree of substitution of the polysaccharide becomes 1.4, which is the average value. This degree of substitution may be identified through $^1$H NMR analysis of the polysaccharide. Since the hydroxyl groups and substituted functional groups present in the polysaccharide can be quantified through the $^1$H NMR analysis, the degree of substitution can be identified, and the degree of substitution can be calculated in consideration of the $^1$H NMR analysis results of the polysaccharide before modification, if necessary.

A method of performing the $^1$H NMR analysis is described in "4. NMR Analysis" of the Example sections in this specification, and an example of calculating the degree of substitution DS according to the results is described in Preparation Example 1 of the Example sections above.

In Equation 1, Mw is the molecular weight of the polysaccharide component, and the molecular weight related to bulkiness is also related to the steric structure associated with the self-cross-linking. The molecular weight is evaluated in the manner described in "2. Measurement of molecular weight of polysaccharide component" of the Example sections in this specification.

Through adjustment of F in Equation 1, which is determined by these factors, it is possible to obtain a polysaccharide component in which the self-cross-linking is effectively performed.

The lower limit of F in Equation 1 may be 2, 2.5, 3. 3.5, 4, 4.5, 5, 5.5, 6, 6.5, 7, 7.5, 8, 8.5, 9, or 9.5, or so and the upper limit thereof may be 100, 95, 90, 85, 80, 75, 70, 65, 60, 55, 50, 45, 40, 35, 30, 25, 20, 15, 10, 9.5, 9, 8.5, 8, 7.5, 7, 6.5, 6, 5.5, 5, 4.5, 4, 3.5, 3, or 2.5 or so. The F may be within a range of more than or equal to, or more than any one of the above-described lower limits; or within a range of less than or equal to, or less than any one of the above-described upper limits while being more than or equal to, or more than any one of the above-described lower limits.

The polysaccharide component with F in Equation 1 within the above range may exhibit properties capable of exhibiting excellent absorption capacity, and exhibit a steric structure suitable for cross-linking while having the substituents in an amount capable of effective self-cross-linking. In addition, such a polysaccharide component may exhibit excellent absorption capacity after cross-linking.

The ranges of Mw, DS and AP for determining F in Equation 1 may be further adjusted to an appropriate level.

For example, the lower limit of Mw in Equation 1 may be 50,000, 100,000, 150,000, 200,000, 250,000, 300,000, 500,000, 1,000,000, 5,000,000, 10,000,000, 15,000,000, 20,000,000, 25,000,000, 30,000,000, 35,000,000, 40,000,000, 45,000,000, 50,000,000, 55,000,000, 60,000,000, or 64,000,000 or so, and the upper limit thereof may be 10,000,000,000, 9,500,000,000, 9,000,000,000, 8,500,000,000, 8,000,000,000, 7,500,000,000, 7,000,000,000, 6,500,000,000, 6,000,000,000, 5,500,000,000, 5,000,000,000, 4,500,000,000, 4,000,000,000, 3,500,000,000, 3,000,000,000, 2,500,000,000, 2,000,000,000, 1,500,000,000, 1,000,000,000, 950,000,000, 900,000,000, 850,000,000, 800,000,000, 750,000,000, 700,000,000, 650,000,000, 600,000,000, 550,000,000, 500,000,000, 450,000,000, 400,000,000, 350,000,000, 300,000,000, 250,000,000, 200,000,000, 150,000,000, 100,000,000, 95,000,000, 90,000,000, 85,000,000, 80,000,000, 75,000,000, 70,000,000, 65,000,000, 60,000,000, 55,000,000, 50,000,000, 45,000,000, 40,000,000, 35,000,000, 30,000,000, 25,000,000, 20,000,000, 15,000,000, 10,000,000, 9,500,000, 9,000,000, 8,500,000, 8,000,000, 7,500,000, 7,000,000, 6,500,000, 6,000,000, 5,500,000, 5,000,000, 4,500,000, 4,000,000, 3,500,000, 3,000,000, 2,500,000, 2,000,000, 1,500,000, 1,000,000, 950,000, 900,000, 850,000, 800,000, 750,000, 700,000, 650,000, 600,000, 550,000, 500,000, 450,000, 400,000, or 350,000 or so. The Mw may be within a range of more than or equal to, or more than any one of the above-described lower limits; or within a range of less than or equal to, or less than any one of the above-described upper limits while being more than or equal to, or more than any one of the above-described lower limits.

In Equation 1, the lower limit of DS may be 0.4, 0.45, 0.5, 0.55, 0.6, 0.65, 0.7, 0.75, 0.8, 0.85, 0.9, 0.95, 1, 1.05, 1.1, 1.15, 1.2, 1.25, 1.3, 1.35, 1.4, 1.45, 1.5, 1.55, or 1.6 or so, and the upper limit thereof may be 2.5, 2.45, 2.4, 2.35, 2.3, 2.25, 2.2, 2.15, 2.1, 2.05, 2, 1.95, 1.9, 1.85, 1.8, 1.75, 1.7, 1.65, 1.6, 1.55, 1.5, 1.45, 1.4, 1.35, 1.3, 1.25, 1.2, 1.15, 1.1, 1.05, 1, 0.95, 0.9, 0.85, 0.8, 0.75, 0.7, 0.65, 0.6, 0.55, 0.5, 0.45, or 0.4 or so. The DS may be within a range of less than or equal to, or less than any one of the above-described upper limits while being more than or equal to, or more than any one of the above-described lower limits.

In Equation 1, the lower limit of AP may be 0.6, 0.65, 0.7, 0.72, 0.74, 0.76, or 0.78 or so, and the upper limit thereof may be 0.95, 0.9, 0.85, 0.8, or 0.75 or so. The AP may be within a range of less than or equal to, or less than any one of the above-described upper limits while being more than or equal to, or more than any one of the above-described lower limits.

The self-cross-linking of the polysaccharide component may be performed, for example, by using a polysaccharide component having a carboxyl group or a salt of a carboxyl group as the acidic group among the above-described acidic polysaccharide components. Since the polysaccharide itself contains hydroxy groups, the self-cross-linking may be performed by performing an esterification reaction between the hydroxy group contained in one molecule of polysaccharide and the carboxyl group or salt of the carboxyl group contained in the acidic polysaccharide.

The type of polysaccharide component having the carboxyl group or salt of the carboxyl group is not particularly limited. For example, the self-cross-linking may be performed by applying polysaccharides having carboxyl groups and the like on their own, such as so-called CMC (carboxylmethyl cellulose), which corresponds to a lignocellulosic polysaccharide, or polysaccharides to which carboxyl groups or salts thereof are introduced through processes such as maleation or carboxyalkylation.

The self-cross-linked polysaccharide component may comprise polymer chains (i.e., polysaccharide chains to be cross-linked) containing monosaccharide units linked by glycosidic bonds, and cross-linking bonds linking the polymer chains.

At this time, the cross-linking bond may be connected to the monosaccharide unit.

Here, the cross-linking bond may be a bond represented by Formula 1 below.

[Formula 1]

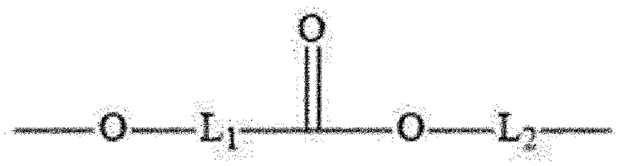

In Formula 1, $L_1$ is an alkylene group, an alkylidene group, or a bond of Formula 2 below, and $L_2$ is represented by a single bond or $-(CH_2)-O-$.

[Formula 2]

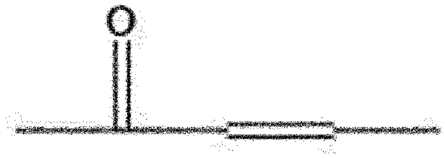

In Formula 1, the oxygen atom shown on the leftmost side may be directly connected to the monosaccharide unit of the polymer chain.

In Formula 1, when $L_2$ is a single bond, the $L_2$ does not exist. That is, in Formula 1, when $L_2$ exists, $L_2$ may be directly connected to the monosaccharide unit of the polymer chain, and when $L_2$ is a single bond, the oxygen atom on the left side of $L_2$ in Formula 1 may be directly connected to the monosaccharide unit.

In Formula 1, the alkylene group means a functional group in which two hydrogen atoms are separated from an alkane and connected to another object, where the two hydrogen atoms are separated from other carbon atoms of the alkane. Such an alkylene group may be an alkylene group with 2 to 20 carbon atoms, 2 to 16 carbon atoms, 2 to 12 carbon atoms, 2 to 8 carbon atoms, or 2 to 4 carbon atoms. Such an alkylene group may be linear, branched, or cyclic. Such an alkylene group may also be optionally substituted with one or more substituents.

In Formula 1, the alkylidene group means a functional group in which two hydrogen atoms are separated from an alkane and connected to another object, where it means a structure in which the two hydrogen atoms are separated from one carbon atom of the alkane. Such an alkylidene group may be an alkylidene group with 1 to 20 carbon atoms, 1 to 16 carbon atoms, 1 to 12 carbon atoms, 1 to 8 carbon atoms, or 1 to 4 carbon atoms. Such an alkylidene group may be linear, branched, or cyclic. Such an alkylidene group may also be optionally substituted with one or more substituents.

In the case where the self-cross-linking of the polysaccharide is performed by a polysaccharide having a carboxyl group or a salt thereof on its own, such as CMC (carboxylmethyl cellulose), or a polysaccharide having a carboxyl group or a salt thereof introduced by carboxyalkylation or the like, $L_1$ in Formula 1 may usually be an alkylene group or an alkylidene group. In Formula 1, when $L_1$ is a functional group of Formula 2, it is the case where the self-cross-linking is performed by a carboxyl group or a salt thereof introduced by so-called maleation or the like.

In Formula 1, when $L_1$ is Formula 2, the carbon atom of the carbonyl group in Formula 2 is connected to the oxygen atom on the left side of $L_1$ in Formula 1, and the carbon atom on the right side of the carbon-carbon double bond in Formula 2 is connected to the carbon atom of the carbonyl group on the right side of $L_1$ in Formula 1.

One or more bonds of Formula 1 may exist within the self-cross-linked polysaccharide component.

The type of the monosaccharide unit is not particularly limited, which may be typically a monosaccharide unit constituting a polysaccharide. For example, such a monosaccharide unit may be a glucose unit, a glucosamine unit, an N-acetylglucosamine unit, and the like.

Such a monosaccharide unit usually contains a ring structure containing carbon atoms and oxygen atoms as ring constituent atoms. The ring structure of the monosaccharide unit is usually a hexa-cyclic ring structure (in the case where the ring atoms include only 5 carbon atoms and 1 oxygen atom), but may also have a ring structure of 6 rings or more. When the ring structure is six rings or more, the ring atoms may be carbon atoms, or heteroatoms such as oxygen or nitrogen atoms.

In the case of the self-cross-linking structure, the bond of Formula 1 above may be directly connected to the carbon atom of the ring structure of the monosaccharide unit, or may be connected via a methylene group ($-CH_2-$).

At least one of or both the leftmost oxygen atom and the rightmost oxygen atom (if $L_2$ is a single bond, it means the oxygen atom connected to the right side of the carbonyl group, and if it is not a single bond, it means the oxygen atom of $-(CH_2)-O-$) in Formula 1 may be directly connected to the carbon atom of the ring structure, or may be connected via the methylene group ($-CH_2-$).

Here, the matter of being connected via the methylene group ($-CH_2-$) is the case where only the methylene group ($-CH_2-$) exists between the leftmost oxygen atom of Formula 1 above or the rightmost oxygen atom (if $L_2$ is a single bond, it means the oxygen atom connected to the right side of the carbonyl group, and if it is not a single bond, it means the oxygen atom of $-(CH_2)-O-$) of Formula 1 and the carbon atom of the ring structure.

In this case, more specifically, the polysaccharide component may contain a unit represented by Formula 3 below.

[Formula 3]

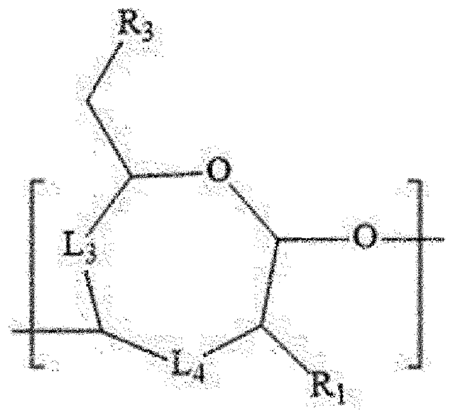

In Formula 3, $R_1$ is a hydroxy group, an amino group, $-L_5-C(=O)-OH$, $-L_5-C(=O)-O^-$, or a functional group of Formula 4 below, $R_3$ is a hydroxy group, $-L_5-C(=O)-OH$, $-L_5-C(=O)-O^-$ or a functional group of Formula 4 below, and one of $L_3$ and $L_4$ is a single bond, the other is $CHR_2$, where $R_2$ is a hydroxy group, $-L_5-C\ (=O)-OH$, $-L_5-C(=O)-O^-$ or a functional group of Formula 4 below, and $L_5$ is an alkylene group or an alkylidene group, but any one of $R_1$ to $R_3$ is the oxygen atom (excluding the oxygen atom present in the carbonyl group) of the bond of Formula 1 above.

[Formula 4]

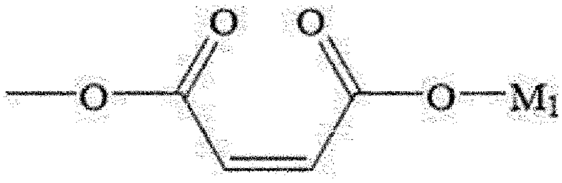

In Formula 4, $M_1$ is hydrogen or a metal, and when $M_1$ is the metal, the bond of $O-M_1$ above is an ionic bond.

The functional group of $-L_5-C(=O)-OH$ or $-L_5-C(=O)-O^-$ in Formula 3 may be, for example, a carboxyl group introduced by carboxyalkylation or a functional group in which the carboxyl group is ionized, and the like, and Formula 4 is a functional group introduced by malation.

In Formula 3, the specific types of alkylene group and alkylidene group are the same as those in Formula 1.

These functional groups are introduced to form cross-linking bonds by participating in the above-described self-cross-linking reaction, but all the introduced functional groups may not participate in the cross-linking reaction, and in this case, some functional groups may remain.

Here, the matter that any one of $L_3$ and $L_4$ is a single bond means that any one of $L_3$ and $L_4$ does not exist. For example, $L_3$ is not present, and the carbon atoms connected to the left and right sides of $L_3$ in Formula 3 are directly connected, and $L_4$ is not present, and the carbon atoms connected to the left and right sides of $L_4$ in Formula 3 are directly connected.

The matter that the other of $L_3$ and $L_4$ is $CHR_2$ means the case where in Formula 3, any one of $L_3$ and $L_4$ is a carbon atom, and the substituent $R_2$ is substituted on the carbon atom.

The matter that any one of $R_1$ to $R_3$ in Formula 3 is the oxygen atom (excluding the oxygen atom present in the carbonyl group) of the bond of Formula 1 means that any one of $R_1$ to $R_3$ is the oxygen atom of the bond of Formula 1 connecting the polysaccharide, where the oxygen atom means any one of the leftmost oxygen atom and the rightmost oxygen atom (if $L_2$ is a single bond, it means the oxygen atom connected to the right side of the carbonyl group, and if it is not a single bond, it means the oxygen atom of $-(CH_2)-O-$) in Formula 1.

As described above, the self-cross-linking structure may be implemented by performing an esterification reaction between acidic polysaccharides, especially, polysaccharides having a carboxyl group or a salt thereof. In such a self-cross-linking reaction, by particularly adjusting the pH among the reaction conditions, as described below, it is possible to provide a polymer having balanced absorption properties.

As the acidic polysaccharides, polysaccharides having a carboxyl group or a salt thereof on their own, such as CMC (carboxylmethyl cellulose), may be used, or polysaccharides in which a carboxyl group or a salt thereof is introduced through a process such as maleation or carboxyalkylation may be used, where to achieve the above-mentioned degree of substitution and for efficient self-cross-linking, polysaccharides into which a carboxyl group or a salt thereof has been introduced through a denaturation process may be used.

The method of introducing a carboxyl group or a salt thereof into a polysaccharide is not particularly limited. For example, to introduce a functional group such as Formula 4, a so-called maleation process may be performed. Such a process is a process of reacting a polysaccharide with an unsaturated dicarboxylic acid or its anhydride to replace the hydroxy group present in the unitary body of the polymer with the functional group, where an example of the dicarboxylic acid or its anhydride may be exemplified by maleic acid or maleic anhydride, and the like, but is not limited thereto, and salts of the maleic acid, and the like may also be applied. The method of performing the maleation process is known.

The carboxyalkylation process may be performed by reacting the polysaccharide component with an alkanoic acid or haloalkanoic acid or a salt of the alkanoic acid or haloalkanoic acid. For example, after protonating the hydroxy group of the polysaccharide component through an additive such as NaOH, a carboxyl group or a salt thereof may be introduced into the polysaccharide component through a reaction with the alkanoic acid.

As is known, the alkanoic acid is an aliphatic acid derived from an alkane, and the haloalkanoic acid means one in which at least one of hydrogen atoms of the alkanoic acid is replaced with a halogen atom (e.g., chlorine, fluorine, or bromine, etc.). In this specification, the alkanoic acid, haloalkanoic acid, salt of alkanoic acid, and/or salt of haloalkanoic acid applied in the carboxyalkylation process may be referred to as a treatment agent.

As the alkanoic acid or haloalkanoic acid used as the treatment agent, an alkanoic acid or haloalkano acid with 1 to 20 carbon atoms, 1 to 16 carbon atoms, 1 to 12 carbon atoms, 1 to 8 carbon atoms, or 1 to 4 carbon atoms may be used, and acetic acid or chloroacetic acid may be typically used.

The salt of the haloalkanoic acid or alkanoic acid may be an alkali metal salt or alkali earth metal salt of the haloalkanoic acid or alkanoic acid having the same number of carbon atoms as above.

An acidic group (carboxyl group, etc.) may be introduced into the polysaccharide by reacting the treatment agent with the polysaccharide under appropriate conditions.

The process of introducing the acidic group may be performed according to a known method, and if necessary, an additional process may be performed or the conditions of the process may be adjusted for efficient progress of the carboxyalkylation process.

For example, the above process may be performed on a mixture in which the treating agent and a hydroxide are dispersed in a solvent. Here, as the solvent, for example, an aqueous solvent such as water may be used. Here, as the water, tap water, distilled water, deionized water, or purified water, and the like may be used. Here, as the hydroxide, ammonium hydroxide or a metal hydroxide may be used, and as the metal hydroxide, sodium hydroxide, potassium hydroxide, or lithium hydroxide, and the like may be used, without being limited thereto.

Such a metal hydroxide can act as the additive protonating the hydroxyl group of the polysaccharide component.

In the reaction to the mixture, for example, a gelatinization reaction of polysaccharides first proceeds within the mixture, and subsequently introduction of carboxyl groups and the like by the treatment agent proceeds.

In the above process, the degree of substitution may be controlled by controlling the amount of the treatment agent in the mixture. In the above reaction, about 90 wt% or so of the generally applied treatment agent reacts with the polysaccharide component, whereby carboxyl groups, and the like are introduced into the polysaccharide component. Therefore, in consideration of this, the desired degree of substitution can be achieved by applying an appropriate level of treatment agent relative to the used polysaccharide component. To achieve a degree of substitution above a certain level, the preparation and reaction of the mixture can be repeated multiple times, and if necessary, the ratio of the hydroxide applied together can also be controlled together.

For example, the lower limit of the ratio of the hydroxide in the mixture may be 0.5 equivalents, 0.6 equivalents, or 0.7 equivalents or so, and the upper limit thereof may be 1.5 equivalents, 1.15 equivalents, 1.1 equivalents, 1 equivalent, 0.9 equivalents, or 0.8 equivalents or so. The equivalent may be within a range of less than or equal to, or less than any one of the above-described upper limits; or within a range of less than or equal to, or less than any one of the above-described upper limits while being more than or equal to, or more than any one of the above-described lower limits. Here, the equivalent can be obtained by an equation A/B, wherein A is the mole number of the hydroxide present in the mixture, and B is a value calculated as C/162.14, where C is the weight (unit: g) of the polysaccharide in the mixture. Here, 162.14 is the molar mass (g/mol) of anhydroglucose unit. Typically, the polysaccharide contains the anhydroglucose unit or a derivative thereof, or a unit with a similar molar mass. Therefore, in the present application, if the used amount of polysaccharide for obtaining the above equivalent is representatively applied by the equation C/162.14, the reaction may proceed according to the purpose by specifying the equivalent according to such an applying manner in the above range. By maintaining the used amount of hydroxide in the above range, it is possible to stably maintain the reaction efficiency and workability while maintaining the substitution efficiency of carboxyl groups, and the like in the desired range, and it is possible to suppress unnecessary side reactions, and the like.

The lower limit of the ratio of the treatment agent in the mixture may be 0.5 equivalents, 0.6 equivalents, or 0.7 equivalents or so, and the upper limit thereof may be 1.5 equivalents, 1.15 equivalents, 1.1 equivalents, 1 equivalent, 0.9 equivalents, or 0.8 equivalents or so. The equivalent may be within a range of less than or equal to, or less than any one of the above-described upper limits; or within a range of less than or equal to, or less than any one of the above-described upper limits while being more than or equal to, or more than any one of the above-described lower limits. Here, the

equivalent can be obtained by an equation D/B, wherein D is the mole number of the treatment agent present in the mixture, and B is the same as the equation for calculating the equivalent of the hydroxide. By maintaining the used amount of the treatment agent in the above range, it is possible to stably maintain the reaction efficiency and workability while maintaining the substitution efficiency of carboxyl groups, and the like in the desired range, and it is possible to suppress unnecessary side reactions, and the like.

The lower limit of the ratio (A/B) of the mole number (A) of the hydroxide and the mole number (D) of the treatment agent in the mixture may be 0.5, 0.6, 0.7, 0.8, 0.9, or 0.95 or so, and the upper limit thereof may be 1.5, 1.4, 1.3, 1.2, 1.1, or 1.05 or so. The ratio may be within a range of less than or equal to, or less than any one of the above-described upper limits; or within a range of less than or equal to, or less than any one of the above-described upper limits while being more than or equal to, or more than any one of the above-described lower limits. By maintaining the above ratio, it is possible to obtain a polysaccharide capable of stable self-cross-linking while maintaining the substitution efficiency of carboxyl groups, and the like in the desired range, it is possible to stably maintain the reaction efficiency and workability, and it is possible to suppress unnecessary side reactions, and the like.

In the above reaction, the mixture may be present in a predetermined ratio in a reactor where the reaction occurs. For example, the lower limit of the ratio of the volume of the mixture in the reactor based on the total volume of the reactor may be 70%, 75%, 76%, or 77% or so, and the upper limit thereof may be 95%, 94%, 93%, or 92% or so. The ratio may be within a range of less than or equal to, or less than any one of the above-described upper limits; or within a range of less than or equal to, or less than any one of the above-described upper limits while being more than or equal to, or more than any one of the above-described lower limits. By maintaining the above ratio, it is possible to stably maintain torque in the reactor, it is possible to obtain a polysaccharide capable of stable self-cross-linking while maintaining the substitution efficiency of carboxyl groups, and the like in the desired range, it is possible to stably maintain the reaction efficiency and workability, and it is possible to suppress unnecessary side reactions, and the like.

The carboxyalkylation may be performed by a method of maintaining the mixture at an appropriate temperature for a constant time or more, and the desired acidic polysaccharide may be obtained. In this process, additional processes such as stirring may be performed, if necessary.

This specification also discloses a method for producing the polymer through a step of self-cross-linking an acidic polysaccharide component, for example, the above-described acidic polysaccharide component.

As the acidic polysaccharide component, the above-described components may be used. Immediately after introducing an acidic group such as a carboxyl group into the polysaccharide component through the above-described reaction, it is also possible to perform the self-cross-linking process, and if necessary, after recovering the polysaccharide component once, it is possible to perform the self-cross-linking process. The recovering of the polysaccharide component may comprise a process of dissolving the reactant resulting from the reaction in water and precipitating it using an organic solvent such as alcohol, and in addition to this, various methods may be applied.

The acidic polysaccharide component may have the range of F in Equation 1 and the values of AP, DS, and Mw in Equation 1 as described above.

The method of performing the self-cross-linking is not particularly limited, but for efficient self-cross-linking, it may be performed by a method of dispersing the acidic polysaccharide in a solvent, and then maintaining the pH within a predetermined range.

In the above process, an aqueous solvent, for example, water may also be used as the solvent, and tap water, distilled water, deionized water, or purified water, and the like may be used as the water.

Even upon the self-cross-linking, it may be appropriate to substantially use only the aqueous solvent (e.g., water) as the solvent. Therefore, the solvent used upon the self-cross-linking may not substantially contain other solvents rather than the aqueous solvent (e.g., water). At this time, the matter that other solvents are not substantially included may mean the case where the upper limit of the content of other solvents rather than the aqueous solvent (for example, water) in the solvent is 10 wt%, 9 wt%, 8 wt%, 7 wt%, 6 wt%, 5 wt%, 4 wt%, 3 wt%, 2 wt%, 1 wt%, 0.5 wt%, 0.1 wt%, 0.05 wt%, 0.01 wt%, 0.005 wt%, or 0.001 wt% or so, and the lower limit thereof is 0 wt% or so. The ratio may be within a range of less than or equal to, or less than any one of the above-described upper limits; or within a range of less than or equal to, or less than any one of the above-described upper limits while being more than or equal to, or more than any one of the above-described lower limits.

The amount of the applied solvent in the above process may be an amount of 5 times to 15 times the weight of the applied polysaccharide. The dissolution of the polysaccharide into the solvent may be performed under room temperature and normal pressure conditions, but is not limited thereto.

The self-cross-linking may proceed by dissolving the polysaccharide in the solvent and maintaining the pH at a constant level. If necessary, additional processes, such as a stirring process, capable of promoting the self-cross-linking may also be performed.

The lower limit of pH maintained in the above process may be 6, 6.1, 6.2, 6.3, 6.4, or 6.5 or so, and the upper limit thereof may be 7, 6.9, 6.8, 6.7, 6.6, 6.5, 6.4, 6.3, 6.2, 6.1, or 6 or so. The pH may be within a range of less than or equal to, or less than any one of the above-described upper limits; or within a range of more than or equal to, or more than any one of the

above-described lower limits; or within a range of less than or equal to, or less than any one of the above-described upper limits while being more than or equal to, or more than any one of the above-described lower limits. By maintaining this pH range, it is possible to effectively perform the desired self-cross-linking.

The method of maintaining pH in the above range is not particularly limited. For example, if the pH in the above range is achieved by adding an acidic polysaccharide, the self-cross-linking may proceed in that state, and if the desired pH is not achieved, the pH may be adjusted by adding an appropriate acid or base in consideration of the desired pH. At this time, for example, the hydroxide applied in the carboxyalkylation may be used as the base, and hydrochloric acid or sulfuric acid, and the like may be used as the acid, without being limited thereto.

In the above reaction process, a catalyst may be added as needed. For example, an ester catalyst promoting the reaction of carboxyl groups and hydroxy groups may be added. As such a catalyst, 4-methylaminopyridine, magnesium acetate, tetra-n-butyl titanate, lead acetate, sodium acetate, potassium acetate, antimony trioxide and/or N-methylimidazole, and the like may be used, without being limited thereto. The catalyst may be added in a catalytic amount and, for example, may be used in a ratio within a range of 0.1 mol to 5 mol relative to 1 mol of the polysaccharide applied to the reaction. The lower limit of the catalyst usage ratio may be 0.1 mol, 0.5 mol, 1 mol, or 2 mol or so, and the upper limit thereof may be 5 mol, 4.5 mol, 4 mol, or 3.5 mol or so. The ratio may be within a range of less than or equal to, or less than any one of the above-described upper limits; or within a range of less than or equal to, or less than any one of the above-described upper limits while being more than or equal to, or more than any one of the above-described lower limits.

The reaction may also be performed in the presence of a heat stabilizer, if necessary. As the applicable heat stabilizer, an organic or inorganic phosphorus compound such as phosphoric acid, an organic ester of phosphoric acid, phosphorous acid or an organic ester of phosphorous acid may be used, and for example, phosphoric acid, alkyl phosphate or aryl phosphate, and the like, which is commercially known as the heat stabilizer, may be used.

The reaction may also be performed in the presence of additives such as thickeners, plasticizers, preservation stabilizers, and/or antioxidants, if necessary.

The cross-linking reaction may be performed at a predetermined temperature. For example, the lower limit of the temperature at which the reaction proceeds may be 80°C, 85°C, 90°C, 95°C, 100°C, 105°C, 110°C, 115°C, or 120°C or so, and the upper limit thereof may be 300°C, 280°C, 260°C, 240°C, 220°C, 200°C, 180°C, 160°C, 140°C, 130°C, or 125°C or so. The temperature may be within a range of less than or equal to, or less than any one of the above-described upper limits while being more than or equal to, or more than any one of the above-described lower limits. The reaction temperature may be achieved by a method, such as hot air supply, infrared irradiation, microwave irradiation, or ultraviolet irradiation.

The time for the reaction to proceed is not particularly limited. For example, the lower limit of the reaction time may be 30 minutes, 60 minutes, 90 minutes, 120 minutes, 150 minutes, or 180 minutes or so, and the upper limit thereof may be 500 minutes, 480 minutes, 460 minutes, 440 minutes, 420 minutes, 400 minutes, 380 minutes, 360 minutes, 340 minutes, 320 minutes, 300 minutes, 280 minutes, 260 minutes, 240 minutes, 220 minutes, 200 minutes, or 180 minutes or so. The time may be within a range of less than or equal to, or less than any one of the above-described upper limits while being more than or equal to, or more than any one of the above-described lower limits.

The desired self-cross-linked polysaccharide component can be obtained by the above method.

After such a cross-linking process, known additional processes may be performed as needed, and for example, processes, such as a process of recovering the self-cross-linked polysaccharide component, a drying process, a grinding process, and/or a surface treatment for the polysaccharide, may be performed.

The polymer may comprise the polysaccharide component as described above, and may further comprise other components, if necessary. Examples of other components which may be included together with the polysaccharide are not particularly limited, but may be exemplified by additional treatment agents for the polysaccharide component, or polymers different from the polysaccharide component, and the like.

For example, the polymer is in the form of powder, and may further comprise a surface treatment agent bonded to the surface of the powder, and the like. Such a surface treatment agents can contribute to ensuring absorption capacity under pressure, and the like by improving the gel strength of the powder.

Since the polymer exhibits excellent absorbency and biodegradability simultaneously, it may be used for various applications.

For example, the polymer may be used as sanitary products such as diapers or sanitary napkins, or absorbent materials applied to other applications requiring absorption. If necessary, further cross-linking, surface treatment, or physical grinding processes may also be performed on the polymer to increase the efficiency for use as the sanitary products or absorbent materials.

Therefore, this specification discloses an absorbent material or sanitary product (for example, diapers, sanitary napkins, etc.) comprising the polymer.

The specific method of forming the absorbent material or sanitary product by applying the polymer is not particularly limited, and for example, the method of forming the absorbent material or sanitary product by applying the existing SAP may be used in the same manner.

## Advantageous Effects

The present specification discloses a polymer. The polymer may be an absorbent polymer used for forming a so-called SAP. Through adjustment of materials applied to a cross-linking process for forming the SAP, and conditions of the cross-linking process, such a polymer may be formed even without using a so-called cross-linking agent or by minimizing its used amount, thereby exhibiting excellent absorption capacity. The polymer may be formed by cross-linking a biodegradable material, even without using the cross-linking agent or by minimizing the same, thereby exhibiting the maximum biodegradability of the material. The present specification also discloses a use of the polymer.

## Description of Drawings

Figure 1 is a $^1$H NMR spectrum of the acidic polysaccharide of Preparation Example 1.

Figure 2 is a $^1$H NMR spectrum of the acidic polysaccharide of Example 3.

Figure 3 is a $^1$H NMR spectrum of the acidic polysaccharide of Example 4.

Figure 4 is a $^1$H NMR spectrum of the acidic polysaccharide of Example 5.

Figure 5 is a $^1$H NMR spectrum of the acidic polysaccharide of Example 6.

Figure 6 is a $^1$H NMR spectrum of the acidic polysaccharide of Example 7.

Figure 7 is a $^1$H NMR spectrum of the acidic polysaccharide of Example 8.

Figure 8 is a $^1$H NMR spectrum of the acidic polysaccharide of Example 9.

Figure 9 is a $^1$H NMR spectrum of the acidic polysaccharide of Comparative Example 7.

Figure 10 is a $^1$H NMR spectrum of the acidic polysaccharide of Comparative Example 8.

Figure 11 is a $^1$H NMR spectrum of the acidic polysaccharide of Comparative Example 11.

## Mode for Invention

Hereinafter, the polymers, and the like disclosed in this specification will be described in detail through examples and comparative examples, but the scope of the polymers, and the like is not limited by the fallowing examples.

### 1. Evaluation of centrifuge retention capacity (CRC)

CRC (Centrifuge Retention Capacity) was measured according to EDANA (European Disposables and Nonwovens Association) WSP 241.3 regulations. About 0.2 g ($W_0$) of a polymer was placed in a non-woven bag, sealed, and then submerged in a physiological saline solution. As the physiological saline solution, an NaCl aqueous solution with a concentration of 0.9 wt% was used. The state was maintained for 30 minutes or so, water was removed from the bag for 3 minutes under a condition of 250 G using a centrifuge, and the mass (g, $W_2$) of the bag was measured. The same operation was performed on the same non-woven bag containing no polymer, and the mass (g, $W_1$) was measured.
The CRC (g/g) was calculated by substituting the measurement results into Equation A below.
The evaluation was conducted under constant temperature and humidity conditions (23 ± 1°C, relative humidity: 50 ± 10%).

[Equation A]

$$CRC\ (g/g) = \{[W_2(g) - W_1(g)]/W_0(g)\} - 1$$

## 2. Measurement of molecular weight of polysaccharide component

A molecular weight was evaluated using a flow FFF (field-flow fractionation)-MALS (Multiangle Light Scattering) method. The method is a method of measuring the molecular weight of a specimen by connecting MALS, which measures the intensity of scattered light at various angles, as a light scattering detector to the flow FFF. As the flow FFF/MALS equipment, Postnova's AF2000 device was used. To measure a molecular weight, a sample for molecular weight measurement was placed in distilled water at a concentration of 5mg/mL, dissolved in a hydrothermal reactor at a temperature set at 150°C for 6 hours, and filtered (cellulose syringe filter) to prepare the specimen. A 0.1M $NH_4OAc$ (ammonium acetate) aqueous solution was prepared, and used as a mobile phase by filtering it using a solvent clarification system. While the specimen was flowed through an analytical channel (300mm × 60mm × 40mm) under conditions of a detector flow of 0.5mL/min, an injection flow of 0.4mL/min, an injection time of 10min, a cross flow of 0.5mL/min, and a transition time of 1min, the molecular weight was measured.

## 3. Content measurement of amylopectin and amylose

Contents of amylopectin and amylose in a polysaccharide component were evaluated according to the method described in a paper (Potato Research 31 (1988) 241-246).

First, a specimen was prepared by dissolving about 5 mg of a polysaccharide (starch) as a sample in about 1 mL of sterile water (Step 1), and heated to 95°C for about 15 minutes in a constant temperature water bath (Step 2).

Subsequently, about 20 μl of the specimen was placed in a cuvette (Step 3), and about 980μl of an iodine solution was added thereto and mixed (Step 4).

Subsequently, absorbance of the specimen mixed with the iodine solution at wavelengths of 525 nm and 700 nm was measured and recorded, respectively (Step 5). The absorbance was measured using KLAB's OPTIZEN POP model.

About 20 μl of water was placed in another cuvette, 980 μl of the iodine solution was added thereto, and mixed (Step 6). For the solution of Step 6, absorbance at wavelengths of 525 nm and 700 nm was measured and recorded, respectively, in the same manner as in Step 5 (Step 7).

The absorbance obtained in Step 7 was subtracted from the absorbance obtained in Step 5, and the ratio (%) of amylose was confirmed according to Equation E below (Step 8).

[Equation E]

$$PA = 3.039 - \frac{7.154 \times \frac{OD_{700}}{OD_{525}}}{3.048 \times \frac{OD_{700}}{OD_{525}}} - 19.192$$

In Equation E, PA is the ratio (%) of amylose, $OD_{700}$ is the value obtained by subtracting the absorbance at a wavelength of 700 nm measured in Step 7 from the absorbance at a wavelength of 700 nm measured in Step 5, and $OD_{525}$ is the value obtained by subtracting the absorbance at a wavelength of 525 nm measured in Step 7 from the absorbance at a wavelength of 525 nm measured in Step 5.

## 4. NMR analysis

$^1$H-NMR analysis for evaluating a degree of substitution of starch as a polysaccharide component was performed in the following manner. A specimen for $^1$H-NMR analysis was prepared by dissolving the polysaccharide component in water, introducing methanol thereto, followed by stirring the mixture, and then drying it after filtration. 50 mg of the obtained specimen was dissolved in a mixed solvent of 0.75 mL of $D_2O$ and 0.25 mL of $D_2SO_4$ as an NMR measurement solvent, and stirred at 90°C for about 1 hour to prepare a sample. The sample took on a dark yellow color due to the stirring. $^1$H NMR analysis was performed at room temperature (about 25°C) using a Varian Unity Inova (500 MHz) spectrometer with a triple resonance 5 mm probe. The $^1$H NMR analysis was performed using Bruker's avance Neo instrument.

## Preparation Example 1. Preparation of acidic polysaccharide (A)

100 g of starch and 400 mL of IPA (isopropyl alcohol) were introduced into a 500 mL round bottom flask, and

stirred. The starch had a molecular weight of about $6.48 \times 10^7$ g/mol or so, where the weight ratio (AP: AM) of amylose (AM) and amylopectin (AP) was about 79:21 or so. The weight ratio of amylose and amylopectin can be adjusted in the manner shown in Example 16. The external temperature was set to 60°C, and after waiting until temperature equilibrium was achieved, about 39 g of an NaOH aqueous solution with a concentration of about 40 wt% was further introduced thereto, and then 46 g of SMCA (Sodium Monochloro Acetate) was further introduced thereto. It was stirred for 2 hours or more while maintaining a heating state with the temperature set at 60°C. Subsequently, it was cooled at the temperature up to room temperature (about 25°C), and filtered. The solid obtained by filtering was washed several times with an MeOH (methanol) aqueous solution with a concentration of 80 wt%, and dried to prepare an acidic polysaccharide (A).

Figure 1 is a $^1$H NMR spectrum of the polysaccharide component obtained in the above manner. The degree of substitution of this polysaccharide component was evaluated in the following manner. First, the integral sum of peaks of 2.57 ppm, 2.58 ppm, 2.60 ppm, 2.64 ppm, 2.66 ppm, 3.15 ppm, 3.16 ppm, 3.33 ppm, and 3.34 ppm as the peaks in the range of 2.5 ppm to 3.6 ppm of the spectrum was set to be 1. Next, the degree of substitution (2-DS) at carbon 2 was obtained as the integral sum at the peaks of 3.34 ppm and 3.33 ppm (doublet, 0.23), and 2.57 ppm and 2.58 ppm (doublet, 0.20), the degree of substitution (3-DS) at carbon 3 was obtained by dividing the integral value at the 2.39 ppm and 2.41 ppm (0.20) peaks by 2, and the degree of substitution (6-DS) at carbon 6 was obtained by dividing the integral value at the 2.14 ppm and 2.15 ppm (0.19) peaks by 2.

Subsequently, all the above degrees of substitution were added together and the value of 2-DS+3-DS+6-DS was taken as the degree of substitution. The degree of substitution obtained by this method was about 0.6 or so.

**Example 1.**

The acidic polysaccharide component of Preparation Example 1 was dispersed in 10 times the amount of distilled water, and 0.5N HCl aqueous solution was added to adjust the pH of the mixture to about 6.0 or so.

Subsequently, the pH-adjusted mixture was applied to a thickness of about 0.2 to 0.5 cm or so, and heat-treated in an oven at a temperature of 120°C or so for about 3 hours to proceed with a self-cross-linking reaction. Subsequently, the self-cross-linked polymer was pulverized and classified to prepare a polymer in the form of particles with a size ranging from about 150μm to 850 μm or so.

**Example 2.**

A polymer in the form of particles was prepared in the same manner as in Example 1, except that the pH of the mixture was adjusted to about 6.5 or so.

**Comparative Example 1.**

A polymer in the form of particles was prepared in the same manner as in Example 1, except that the pH of the mixture was adjusted to about 4.5 or so.

**Comparative Example 2.**

A polymer in the form of particles was prepared in the same manner as in Example 1, except that the pH of the mixture was adjusted to about 5.0 or so.

**Comparative Example 3.**

A polymer in the form of particles was prepared in the same manner as in Example 1, except that the pH of the mixture was adjusted to about 5.5 or so.

**Comparative Example 4.**

A polymer in particle form was prepared in the same manner as in Example 1, except that the pH of the mixture was adjusted to about 7.0 or so.

**Comparative Example 5.**

A polymer in the form of particles was prepared in the same manner as in Example 1, except that the pH of the mixture was adjusted to about 8.0 or so by using a 0.5N NaOH aqueous solution instead of the 0.5N HCl aqueous solution.

**Comparative Example 6.**

A polymer in the form of particles was prepared in the same manner as in Comparative Example 5, except that the pH of the mixture was adjusted to about 9.0 or so.

The CRC evaluation results of the polymers of Examples and Comparative Examples above were summarized and described in Table 1 below.

[Table 1]

| | CRC (g/g) |
|---|---|
| Example 1 | 34.0 |
| Example 2 | 43.7 |
| Comparative Example 1 | 5.9 |
| Comparative Example 2 | 11.6 |
| Comparative Example 3 | 23.3 |
| Comparative Example 4 | 23.3 |
| Comparative Example 5 | 19.2 |
| Comparative Example 6 | 6.4 |

From the results in Table 1, it can be known that if the pH is too low or high during the self-cross-linking process, a polymer with the desired absorption capacity cannot be obtained. It is determined that if the self-cross-linking reaction promoted in acidic conditions occurs too quickly or too loosely depending on the pH, such a result is due to absorption capacity deterioration, or the occurrence of leakage due to the polymer solution during the absorption process.

**Example 3.**

An acidic polysaccharide was prepared in the same manner as in Preparation Example 1, but the acidic polysaccharide was prepared so that the degree of substitution was about 0.4 or so. The degree of substitution can be adjusted by adjusting the amount of SMCA (Sodium Monochloro Acetate) applied during the preparation process. Typically, about 90 wt% or so of the applied SMCA reacts with starch to introduce carboxyl groups, and in consideration of this, if the amount of the applied SMCA is changed to about 30 g or so, and accordingly, the introduced amount of NaOH aqueous solution (40 wt%) is changed to about 25 g or so, an acidic polysaccharide with a degree of substitution of about 0.4 or so can be produced.

Figure 2 is $^{1}H$ NMR results of the above-prepared acidic polysaccharide, and the degree of substitution obtained in the same manner as in Preparation Example 1 was about 0.4 or so. The self-cross-linking was performed on the acidic polysaccharide in the same manner as in Example 2 to obtain a self-cross-linked polymer.

**Example 4.**

An acidic polysaccharide was prepared in the same manner as Preparation Example 1, but a self-acidic polysaccharide with a degree of substitution of about 0.5 or so was obtained by adjusting the amount of SMCA (Sodium Monochloro Acetate) to about 37 g or so, and accordingly, changing the introduced amount of NaOH aqueous solution (40 wt%) to about 30.5 g. Figure 3 is $^{1}H$ NMR results of the acidic polysaccharide. The self-cross-linking was performed on the acidic polysaccharide in the same manner as in Example 2 to obtain a self-cross-linked polymer.

**Example 5.**

An acidic polysaccharide was prepared in the same manner as Preparation Example 1, but a self-acidic polysaccharide with a degree of substitution of about 0.7 or so was obtained by adjusting the amount of SMCA (Sodium Monochloro Acetate) to about 52 g or so, and accordingly, changing the introduced amount of NaOH aqueous solution (40 wt%) to about 43 g. Figure 4 is $^{1}H$ NMR results of the acidic polysaccharide. The self-cross-linking was performed on the acidic polysaccharide in the same manner as in Example 2 to obtain a self-cross-linked polymer.

**Example 6.**

An acidic polysaccharide was prepared in the same manner as Preparation Example 1, but a self-acidic polysaccharide with a degree of substitution of about 0.8 or so was obtained by adjusting the amount of SMCA (Sodium Monochloro Acetate) to about 58 g or so, and accordingly, changing the introduced amount of NaOH aqueous solution (40 wt%) to about 49 g. Figure 5 is $^1$H NMR results of the acidic polysaccharide. The self-cross-linking was performed on the acidic polysaccharide in the same manner as in Example 2 to obtain a self-cross-linked polymer.

**Example 7.**

An acidic polysaccharide was prepared in the same manner as Preparation Example 1, but a self-acidic polysaccharide with a degree of substitution of about 0.9 or so was obtained by adjusting the amount of SMCA (Sodium Monochloro Acetate) to about 65 g or so, and accordingly, changing the introduced amount of NaOH aqueous solution (40 wt%) to about 55 g. Figure 6 is $^1$H NMR results of the acidic polysaccharide. The self-cross-linking was performed on the acidic polysaccharide in the same manner as in Example 2 to obtain a self-cross-linked polymer.

**Example 8.**

An acidic polysaccharide was prepared in the same manner as Preparation Example 1, but a self-acidic polysaccharide with a degree of substitution of about 1.0 or so was obtained by adjusting the amount of SMCA (Sodium Monochloro Acetate) to about 72 g or so, and accordingly, changing the introduced amount of NaOH aqueous solution (40 wt%) to about 61 g. Figure 7 is $^1$H NMR results of the acidic polysaccharide. The self-cross-linking was performed on the acidic polysaccharide in the same manner as in Example 2 to obtain a self-cross-linked polymer.

**Example 9.**

An acidic polysaccharide was prepared in the same manner as Preparation Example 1, but a self-acidic polysaccharide with a degree of substitution of about 1.2 or so was obtained by adjusting the amount of SMCA (Sodium Monochloro Acetate) to about 99 g or so, and accordingly, changing the introduced amount of NaOH aqueous solution (40 wt%) to about 83 g. Figure 8 is $^1$H NMR results of the acidic polysaccharide. The self-cross-linking was performed on the acidic polysaccharide in the same manner as in Example 2 to obtain a self-cross-linked polymer.

**Example 10.**

An acidic polysaccharide was prepared in the same manner as Preparation Example 1, but a self-acidic polysaccharide with a degree of substitution of about 1.4 or so was obtained by introducing an excess amount (about 120 g or more) of SMCA (Sodium Monochloro Acetate), and accordingly, changing the introduced amount of NaOH aqueous solution (40 wt%) to about 100 g. The self-cross-linking was performed on the acidic polysaccharide in the same manner as in Example 2 to obtain a self-cross-linked polymer.

**Example 11.**

The process of Example 10 of preparing the acidic polysaccharide was repeated twice to obtain a self-acidic polysaccharide with a degree of substitution of about 1.6 or so. The self-cross-linking was performed on the acidic polysaccharide in the same manner as in Example 2 to obtain a self-cross-linked polymer.

**Comparative Example 7.**

An acidic polysaccharide was prepared in the same manner as Preparation Example 1, but a self-acidic polysaccharide with a degree of substitution of about 0.3 or so was obtained by adjusting the amount of SMCA (Sodium Monochloro Acetate) to about 22 g or so. Figure 9 is $^1$H NMR results of the acidic polysaccharide. The self-cross-linking was performed on the acidic polysaccharide in the same manner as in Example 2 to obtain a self-cross-linked polymer.

**Comparative Example 8.**

The process of Example 10 of preparing the acidic polysaccharide was repeated four times to obtain a self-acidic polysaccharide with a degree of substitution of about 2.1 or so. Figure 10 is $^1$H NMR results of the acidic polysaccharide. The self-cross-linking was performed on the acidic polysaccharide in the same manner as in Example 2 to obtain a self-cross-linked polymer.

**Comparative Example 9.**

The process of Example 10 of preparing the acidic polysaccharide was repeated 5 times or more to obtain a self-acidic polysaccharide with a degree of substitution of about 2.5 or so. Figure 11 is $^{1}H$ NMR results of the acidic polysaccharide. The self-cross-linking was performed on the acidic polysaccharide in the same manner as in Example 2 to obtain a self-cross-linked polymer.

**Comparative Example 10.**

A cross-linked polymer was obtained in the same manner as in Comparative Example 9, except that EG (ethylene glycol) was added in an amount of about 0.3 wt% to the mixture during the reaction process for self-cross-linking. In this case, the EG acts as an internal cross-linking agent, and thus the obtained polymer is not a self-cross-linked polymer.

**Comparative Example 11.**

A cross-linked polymer was obtained in the same manner as in Comparative Example 9, except that EG (ethylene glycol) was added to the mixture in an amount of about 0.5 wt% during the reaction process for self-cross-linking. In this case, the EG acts as an internal cross-linking agent, and thus the obtained polymer is not a self-cross-linked polymer.

The CRC evaluation results of the polymers of Examples and Comparative Examples above were summarized and described in Table 2 below.

[Table 2]

| | CRC (g/g) |
|---|---|
| Example 3 | 35.7 |
| Example 4 | 38.4 |
| Example 5 | 45.8 |
| Example 6 | 46.1 |
| Example 7 | 43.4 |
| Example 8 | 42.1 |
| Example 9 | 40.8 |
| Example 10 | 38.2 |
| Example 11 | 34.5 |
| Comparative Example 7 | 28.2 |
| Comparative Example 8 | 19.2 |
| Comparative Example 9 | 10.8 |
| Comparative Example 10 | 24.4 |
| Comparative Example 11 | 24.8 |

From the results in Table 2, it can be known that if the amount of carboxyl groups required for self-cross-linking increases, an overly dense cross-linking structure is realized and simultaneously the ratio of polar functional groups decreases, whereby absorption capacity is lowered. In addition, it can be known that if the amount of the carboxyl group is too small, the cross-linking is not sufficiently achieved, whereby absorption capacity is also lowered. In the case of Comparative Example 9 in which the degree of substitution was not appropriately adjusted, the desired absorption capacity was not obtained even when the internal cross-linking agent (EG) was added.

**Example 12.**

An acidic polysaccharide was prepared in the same manner as in Preparation Example 1, but the acidic polysaccharide was prepared by using, as starch, starch having a molecular weight of about $3.64 \times 10^5$ g/mol or so, and a

weight ratio (AP: AM) of amylose (AM) and amylopectin (AP) of about 79:21 or so, and allowing to have a degree of substitution of about 0.7 or so. The self-cross-linking was performed on the acidic polysaccharide in the same manner as in Example 2 to obtain a self-cross-linked polymer.

**Example 13.**

An acidic polysaccharide was prepared in the same manner as in Preparation Example 1, but the acidic polysaccharide was prepared by using, as starch, starch having a molecular weight of about $8.72 \times 10^6$ g/mol or so, and a weight ratio (AP: AM) of amylose (AM) and amylopectin (AP) of about 79:21 or so, and allowing to have a degree of substitution of about 0.7 or so.

The self-cross-linking was performed on the acidic polysaccharide in the same manner as in Example 2 to obtain a self-cross-linked polymer.

**Example 14.**

An acidic polysaccharide was prepared in the same manner as in Preparation Example 1, but the acidic polysaccharide was prepared by using, as starch, starch having a molecular weight of about $3.29 \times 10^5$ g/mol or so, and a weight ratio (AP: AM) of amylose (AM) and amylopectin (AP) of about 79:21 or so, and allowing to have a degree of substitution of about 0.7 or so.

The self-cross-linking was performed on the acidic polysaccharide in the same manner as in Example 2 to obtain a self-cross-linked polymer.

**Example 15.**

An acidic polysaccharide was prepared in the same manner as in Preparation Example 1, but the acidic polysaccharide was prepared by using, as starch, starch having a molecular weight of about $5.20 \times 10^7$ g/mol or so, and a weight ratio (AP: AM) of amylose (AM) and amylopectin (AP) of about 79:21 or so, and allowing to have a degree of substitution of about 0.7 or so.

The self-cross-linking was performed on the acidic polysaccharide in the same manner as in Example 2 to obtain a self-cross-linked polymer.

The CRC evaluation results of the polymers of Examples above were summarized and described in Table 3 below.

[Table 3]

| | CRC (g/g) |
|---|---|
| Example 12 | 33.5 |
| Example 13 | 38.8 |
| Example 14 | 32.7 |
| Example 15 | 42.1 |

**Example 16.**

An acidic polysaccharide was prepared in the same manner as in Preparation Example 1, except that the weight ratio (AP: AM) of amylose (AM) and amylopectin (AP) was adjusted to about 72:28 or so. The molecular weight of the starch thus adjusted was about $1 \times 10^7$ g/mol, and the degree of substitution of the acidic polysaccharide was about 0.7 or so.

The adjustment of the ratio of amylose and amylopectin in the starch can be performed using n-butanol in the following manner. First, about 10 g of starch, 10 mL of ethanol, and about 300 mL of distilled water are mixed, and stirred at about 90°C until the mixture becomes transparent. Subsequently, 200 mL of a mixture of n-butanol and isoamyl alcohol at a volume ratio of 3:1 (n-butanol: isoamyl alcohol) is introduced to the transparent mixture, and the mixture is further heated for 20 minutes to 30 minutes until the mixture becomes transparent (about 90°C). Subsequently, it is stored at about 4°C for about 24 hours after cooling it up to room temperature (about 25°C), and then centrifuged (3000xg, 20 minutes), and then the separated product is washed with ethanol, and then dried to obtain starch. Since the amylose selectively forms a precipitate with n-butanol, the starch obtained through the above process has an increased amylose content compared to the existing starch. The above process can be repeated until the desired amylose content is obtained.

The self-cross-linking was performed on the acidic polysaccharide in the same manner as in Example 2 to obtain

a self-cross-linked polymer.

**Comparative Example 12.**

An acidic polysaccharide and a self-cross-linked polymer were each prepared as in Example 16, except that starch (waxy corn starch) with a weight ratio (AP: AM) of amylose (AM) and amylopectin (AP) of about 96:4 or so was used.

**Comparative Example 13.**

An acidic polysaccharide and a self-cross-linked polymer were each prepared as in Example 16, except that the weight ratio (AP: AM) of amylose (AM) and amylopectin (AP) was adjusted to about 61:39 or so.

**Comparative Example 14.**

An acidic polysaccharide and a self-cross-linked polymer were each prepared as in Example 16, except that the weight ratio (AP: AM) of amylose (AM) and amylopectin (AP) was adjusted to about 48:52 or so.

**Comparative Example 15.**

An acidic polysaccharide and a self-cross-linked polymer were each prepared as in Example 16, except that the weight ratio (AP: AM) of amylose (AM) and amylopectin (AP) was adjusted to about 40:60 or so.
The CRC evaluation results of the polymers were summarized and described in Table 4 below.

[Table 4]

| | CRC (g/g) |
|---|---|
| Example 16 | 35.8 |
| Comparative Example 12 | 14.6 |
| Comparative Example 13 | 20.2 |
| Comparative Example 14 | 16.6 |
| Comparative Example 15 | 15.7 |

## Claims

1. A polymer comprising a self-cross-linked polysaccharide component, and wherein the polymer has a centrifuge retention capacity of 30 g/g or more according to EDANA (European Disposables and Nonwovens Association) method WSP 241.3.

2. The polymer according to claim 1, wherein the polysaccharide component is starch.

3. The polymer according to claim 1, comprising the self-cross-linked polysaccharide component in an amount of 80 wt% or more.

4. The polymer according to claim 1, wherein the polysaccharide component is an acidic polysaccharide component.

5. The polymer according to claim 1, wherein in Equation 1 below, F is 2.00 or more:

[Equation 1]

$$F = AP \times DS \times \mathrm{Log}\ (Mw)$$

wherein, AP is the ratio of amylopectin in the polysaccharide component, DS is the degree of substitution of the polysaccharide component, and Mw is the molecular weight of the polysaccharide component.

**6.** The polymer according to claim 5, wherein AP in Equation 1 is in a range of 0.7 to 0.9.

**7.** The polymer according to claim 5, wherein DS in Equation 1 is in a range of 0.4 to 2.0.

**8.** The polymer according to claim 5, wherein Mw in Equation 1 is 10,000,000 or more.

**9.** The polymer material according to claim 1, wherein the self-cross-linked polysaccharide component comprises polymer chains containing monosaccharide units linked by glycosidic bonds, and cross-linking bonds of Formula 1 below linking the polymer chains:

[Formula 1]

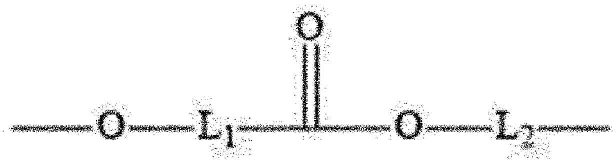

wherein, $L_1$ is an alkylene group, an alkylidene group, or a bond of Formula 2 below, and $L_2$ is represented by a single bond or $-(CH_2)-O-$:

[Formula 2]

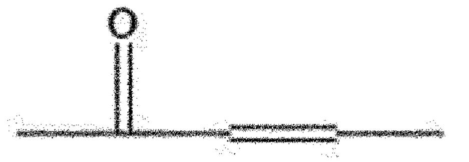

**10.** The polymer material according to claim 9, wherein the monosaccharide unit has a ring structure including carbon atoms and oxygen atoms as ring constituent atoms, and the bond of Formula 1 is directly connected to the carbon atom of the ring structure, or is connected via a methylene group.

**11.** The polymer material according to claim 1, wherein the polysaccharide component contains a unit represented by Formula 3 below:

[Formula 3]

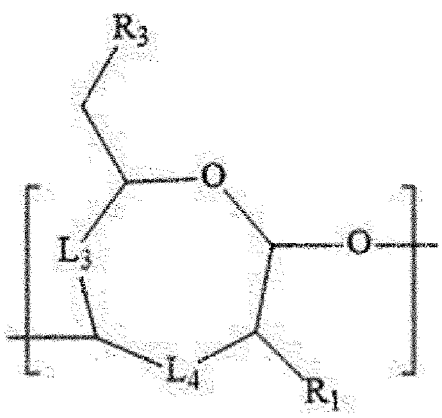

wherein, $R_1$ is a hydroxy group, an amino group, $-L_5-C(=O)-OH$, $-L_5-C(=O)-O^-$, or a functional group of Formula 4 below, $R_3$ is a hydroxy group, $-L_5-C(=O)-OH$, $-L_5-C(=O)-O^-$ or a functional group of Formula 4 below, and one of $L_3$ and $L_4$ is a single bond, the other is $CHR_2$, where $R_2$ is a hydroxy group, $-L_5-C(=O)-OH$, $-L_5-C(=O)-O^-$ or a functional group

of Formula 4 below, and $L_5$ is an alkylene group or an alkylidene group, but any one of $R_1$ to $R_3$ is the oxygen atom (excluding the oxygen atom present in the carbonyl group) of the bond of Formula 1 above:

[Formula 4]

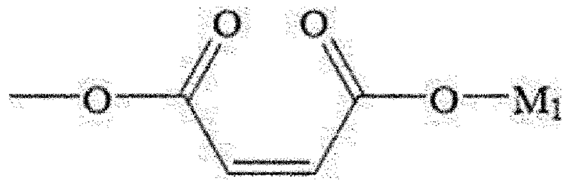

wherein, $M_1$ is hydrogen or a metal, and when $M_1$ is the metal, the bond of O-$M_1$ above is an ionic bond.

**12.** A method for producing the polymer of any one of claims 1 to 11, comprising a step of self-cross-linking an acidic polysaccharide component under a condition where pH is 6 or more and less than 7.

**13.** The method for producing the polymer according to claim 12, wherein the acidic polysaccharide component has F of 2.00 or more in Equation 1 below:

[Equation 1]

$$F = AP \times DS \times \text{Log}\ (Mw)$$

wherein, AP is the ratio of amylopectin in the polysaccharide component, DS is the degree of substitution of the polysaccharide component, and Mw is the molecular weight of the polysaccharide component.

**14.** An absorbent material comprising the polymer of any one of claims 1 to 11.

**15.** A sanitary article comprising the polymer of any one of claims 1 to 11.

[Figure 1]

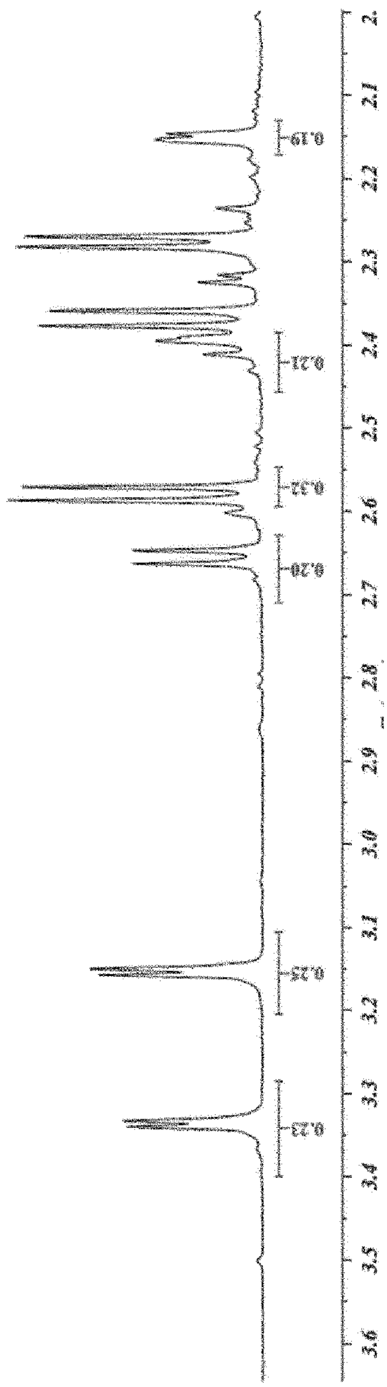
0.19
0.21
0.32
0.20
0.25
0.23
2.
2.1
2.2
2.3
2.4
2.5
2.6
2.7
2.8
2.9
3.0
3.1
3.2
3.3
3.4
3.5
3.6

[Figure 2]

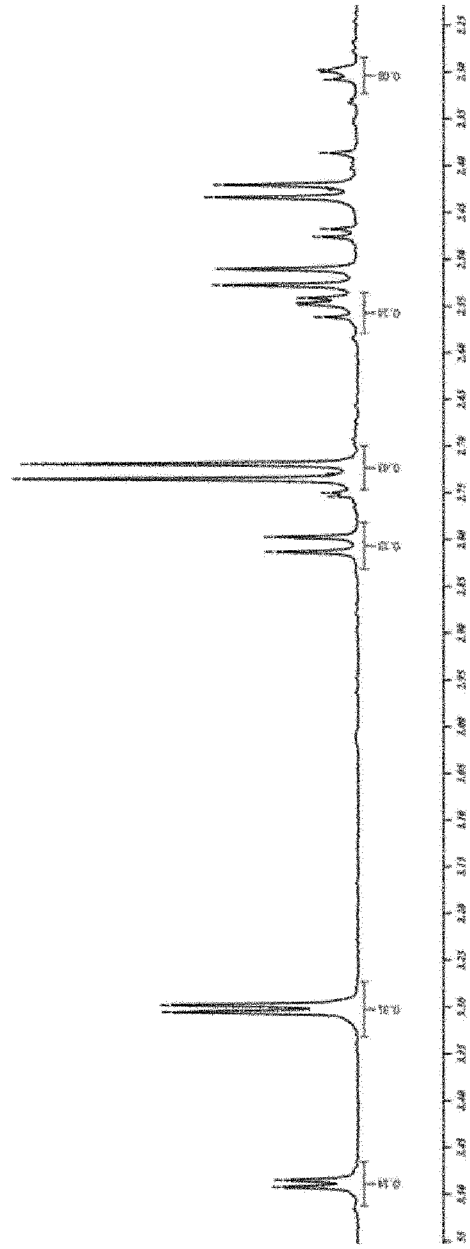

[Figure 3]

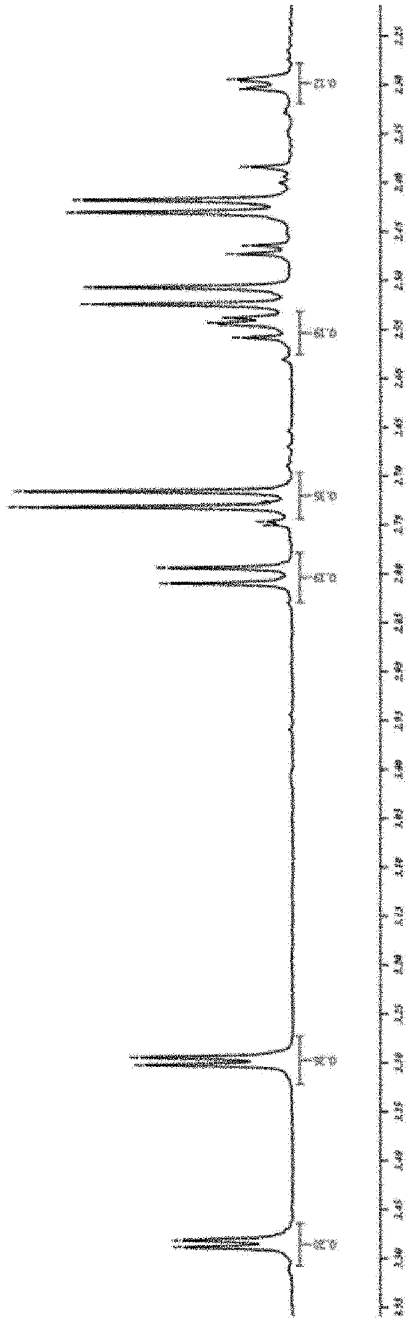

[Figure 4]

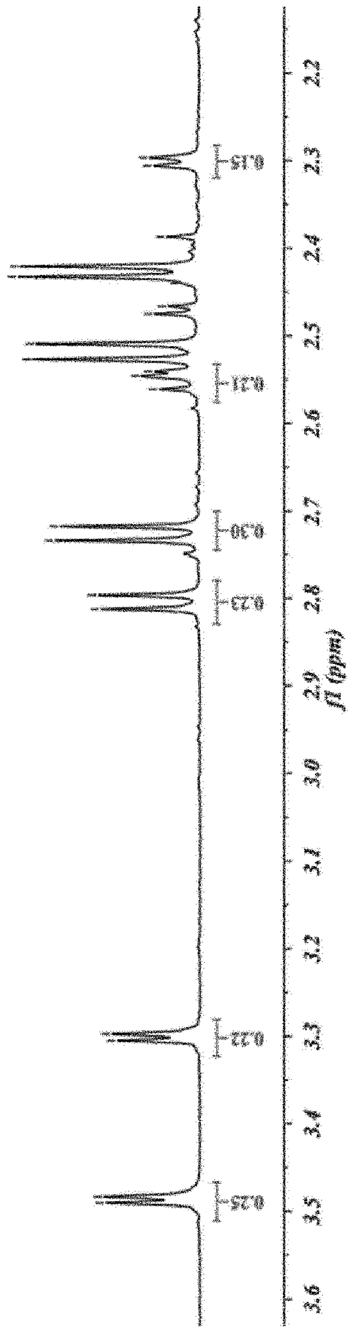
0.15
0.21
0.30
0.23
0.22
0.25
2.2
2.3
2.4
2.5
2.6
2.7
2.8
2.9
f1 (ppm)
3.0
3.1
3.2
3.3
3.4
3.5
3.6

[Figure 5]

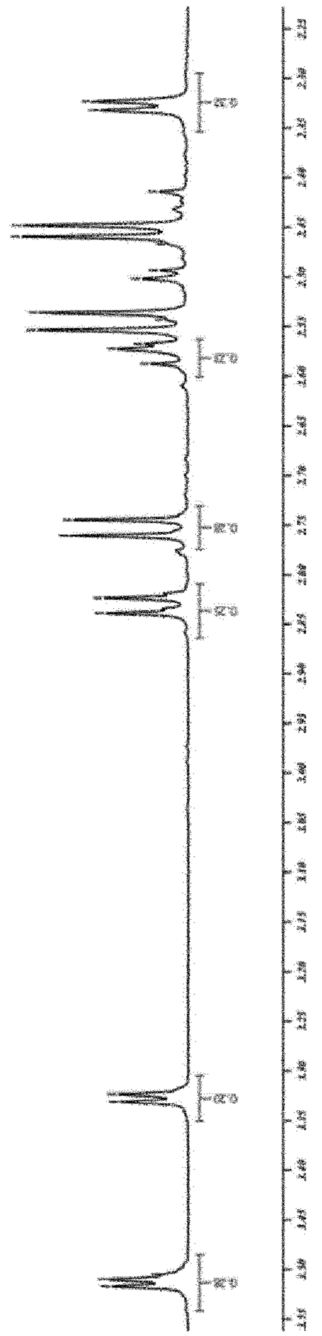

[Figure 6]

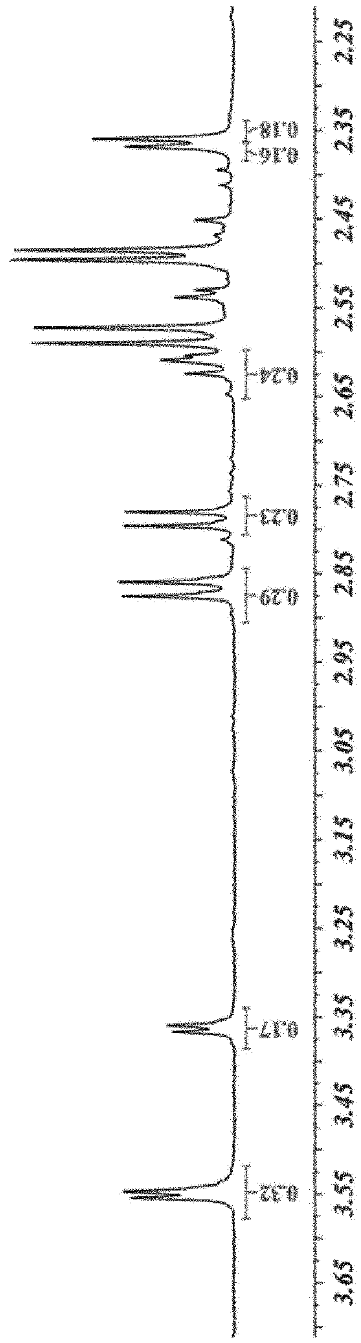
0.18
0.16
0.24
0.23
0.29
0.17
0.32
2.25
2.35
2.45
2.55
2.65
2.75
2.85
2.95
3.05
3.15
3.25
3.35
3.45
3.55
3.65

[Figure 7]

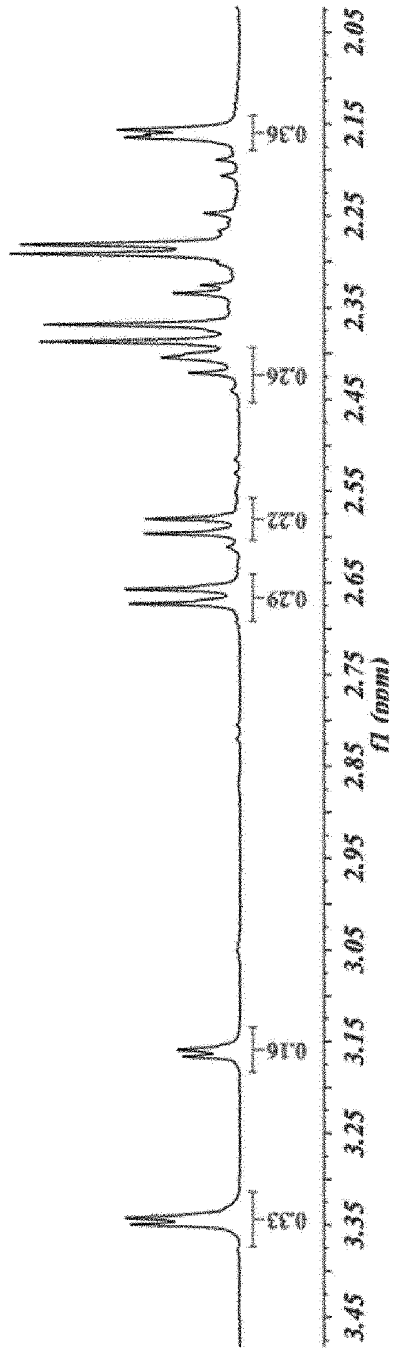
0.36
0.26
0.22
0.29
0.16
0.33
2.05
2.15
2.25
2.35
2.45
2.55
2.65
2.75
2.85
2.95
3.05
3.15
3.25
3.35
3.45
f1 (ppm)

[Figure 8]

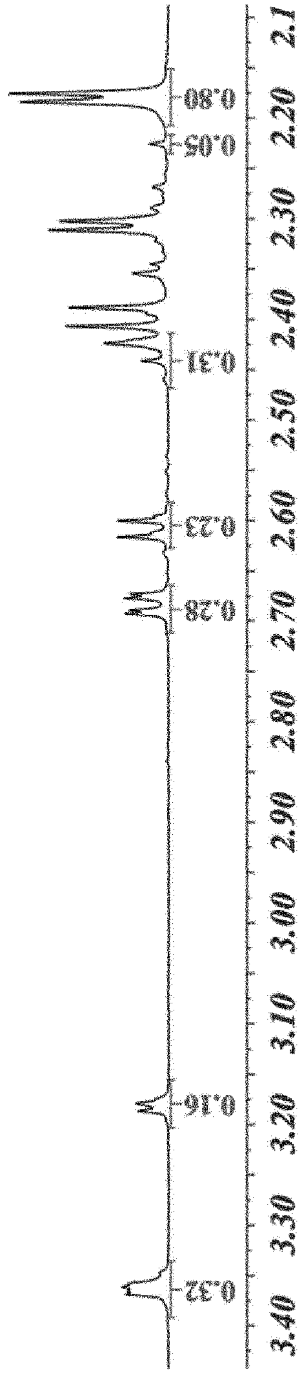
0.80
0.05
0.31
0.23
0.28
0.16
0.32
2.1
2.20
2.30
2.40
2.50
2.60
2.70
2.80
2.90
3.00
3.10
3.20
3.30
3.40

[Figure 9]

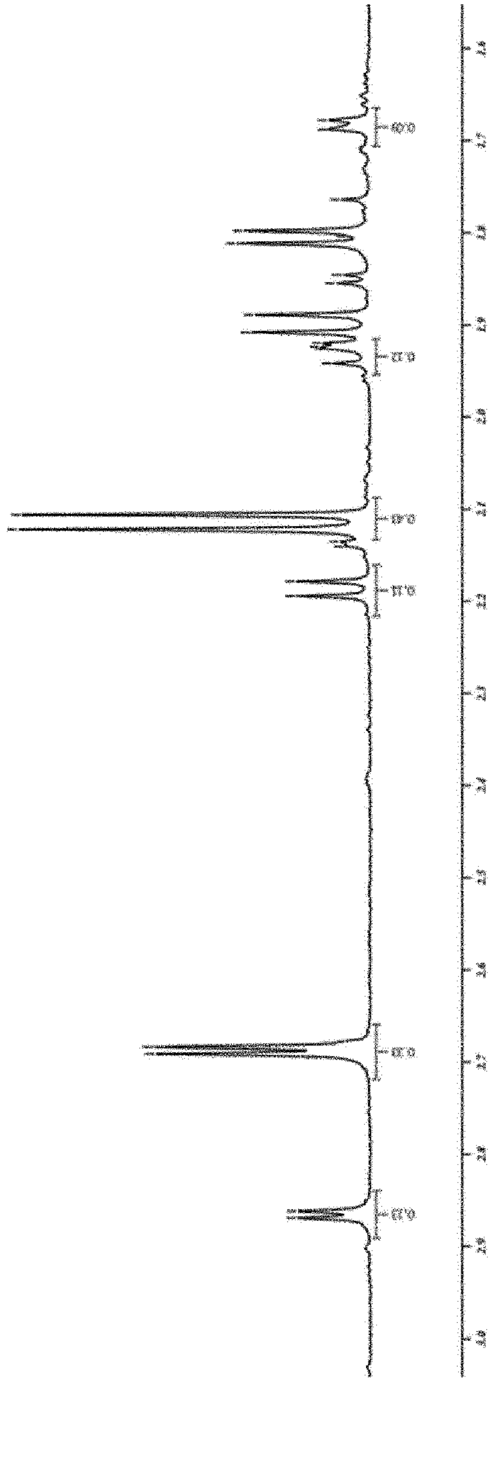

[Figure 10]

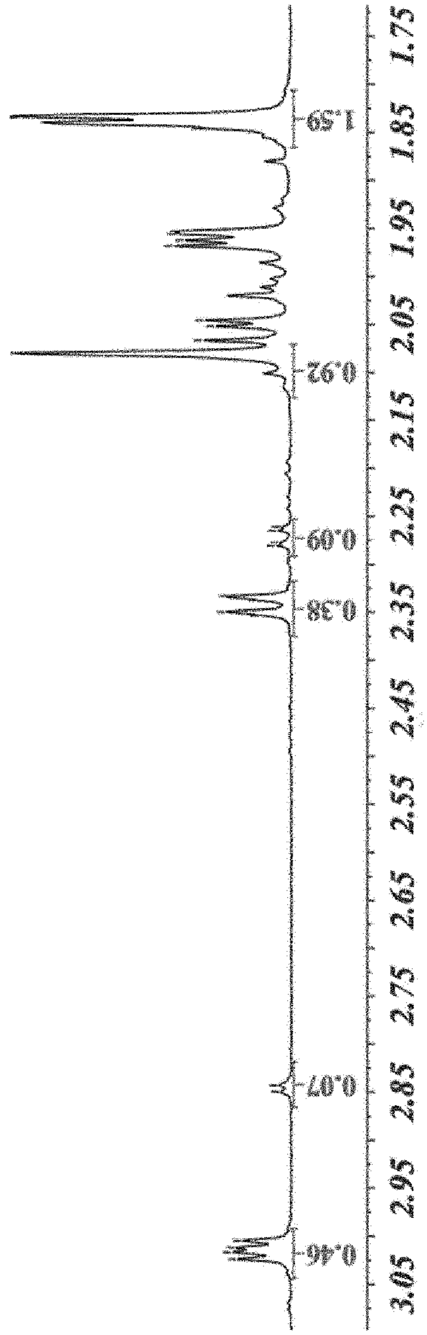
1.75
1.85
1.95
2.05
2.15
2.25
2.35
2.45
2.55
2.65
2.75
2.85
2.95
3.05
1.59
0.92
0.09
0.38
0.07
0.46

[Figure 11]

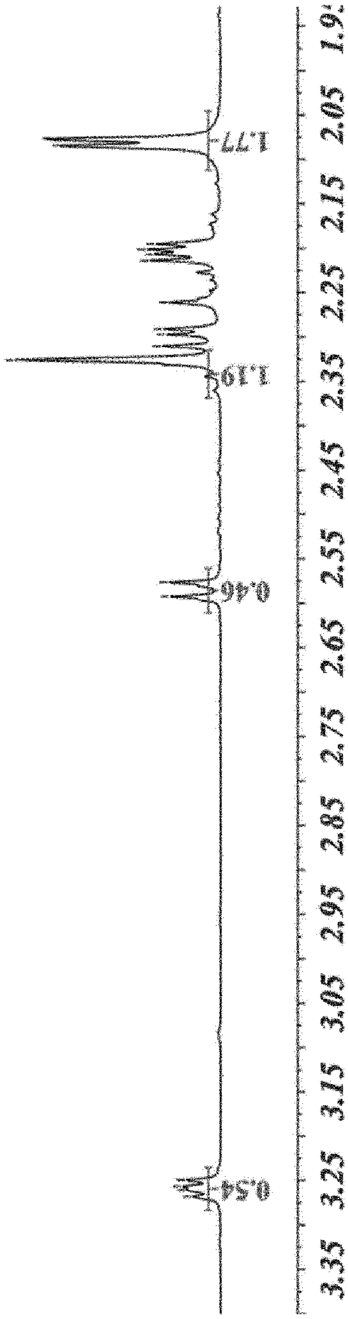
1.77
1.19
0.46
0.54
1.95
2.05
2.15
2.25
2.35
2.45
2.55
2.65
2.75
2.85
2.95
3.05
3.15
3.25
3.35

**INTERNATIONAL SEARCH REPORT**

International application No.
**PCT/KR2023/021369**

**A. CLASSIFICATION OF SUBJECT MATTER**

**C08L 3/14**(2006.01)i; **C08B 30/20**(2006.01)i; **C08B 31/00**(2006.01)i; **A61L 15/22**(2006.01)i

According to International Patent Classification (IPC) or to both national classification and IPC

**B. FIELDS SEARCHED**

Minimum documentation searched (classification system followed by classification symbols)

C08L 3/14(2006.01); C08B 31/10(2006.01); C08J 3/28(2006.01); D06M 15/03(2006.01); D06M 15/263(2006.01); G01N 21/3577(2014.01); G06F 3/14(2006.01); H04M 11/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 자가-가교(self-crosslinking), 다당류 (polysaccharide), 전분(starch), 원심 분리 보수능(centrifuge retention capacity, CRC)

**C. DOCUMENTS CONSIDERED TO BE RELEVANT**

| Category* | Citation of document, with indication, where appropriate, of the relevant passages | Relevant to claim No. |
|---|---|---|
| X | KR 10-1242010 B1 (ARCHER-DANIELS-MIDLAND COMPANY) 13 March 2013 (2013-03-13)<br>See claims 1, 2, 10 and 11; and paragraphs [0012], [0023]-[0027], [0178], [0179] and [0200]-[0202]. | 1-15 |
| A | 이정민. 전분 알데하이드와 카르복시메틸셀룰로오스를 가교시킨 다당류 고흡수성 수지의 제조 및 특성. 서울대학교 대학원 박사학위 논문. 2019, pp. 1-170 (LEE, Jung Min. Preparation and Characterization of Polysaccharide Superabsorbent Polymers Crosslinked with Starch Aldehydes and Carboxymethylcellulose. Ph.D. thesis, The Graduate School, Seoul National University.)<br>See abstract; pages 1 and 107-116; and figures 6 and 20. | 1-15 |
| A | JP 2008-111027 A (JAPAN ATOMIC ENERGY AGENCY) 15 May 2008 (2008-05-15)<br>See claims 1-10. | 1-15 |
| A | JP 2006-081055 A (TERUMO KABUSHIKI KAISHA) 23 March 2006 (2006-03-23)<br>See claims 1-20. | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 April 2024** | **09 April 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
**PCT/KR2023/021369**

**C. DOCUMENTS CONSIDERED TO BE RELEVANT**

| Category* | Citation of document, with indication, where appropriate, of the relevant passages | Relevant to claim No. |
|---|---|---|
| A | JP 2021-519871 A (GREEN POLYMERS LTD.) 12 August 2021 (2021-08-12) See claims 1-13. | 1-15 |

Form PCT/ISA/210 (second sheet) (July 2022)

**INTERNATIONAL SEARCH REPORT**
**Information on patent family members**

International application No.
**PCT/KR2023/021369**

| Patent document cited in search report | Publication date (day/month/year) | Patent family member(s) | Publication date (day/month/year) |
|---|---|---|---|
| KR 10-1242010 B1 | 13 March 2013 | AU 2007-302586 A1 | 03 April 2008 |
| | | AU 2007-302586 B2 | 27 June 2013 |
| | | CA 2664392 A1 | 03 April 2008 |
| | | CN 101541836 A | 23 September 2009 |
| | | CN 101541836 B | 08 April 2015 |
| | | CN 104774275 A | 15 July 2015 |
| | | EP 2066699 A1 | 10 June 2009 |
| | | EP 2066699 B1 | 12 February 2020 |
| | | JP 2010-504414 A | 12 February 2010 |
| | | JP 2013-253262 A | 19 December 2013 |
| | | JP 5765884 B2 | 19 August 2015 |
| | | JP 5947768 B2 | 06 July 2016 |
| | | KR 10-1329658 B1 | 14 November 2013 |
| | | MX 2009003252 A | 02 November 2009 |
| | | MX 339603 B | 31 May 2016 |
| | | US 2008-0177057 A1 | 24 July 2008 |
| | | US 2013-0296548 A1 | 07 November 2013 |
| | | US 8461129 B2 | 11 June 2013 |
| | | WO 2008-037082 A1 | 03 April 2008 |
| JP 2008-111027 A | 15 May 2008 | None | |
| JP 2006-081055 A | 23 March 2006 | JP 4757468 B2 | 24 August 2011 |
| JP 2021-519871 A | 12 August 2021 | CN 112512470 A | 16 March 2021 |
| | | CN 112512470 B | 05 May 2023 |
| | | EP 3773388 A1 | 17 February 2021 |
| | | JP 7384818 B2 | 21 November 2023 |
| | | US 2021-0187477 A1 | 24 June 2021 |
| | | WO 2019-195271 A1 | 10 October 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020220183662


### Non-patent literature cited in the description


- *Potato Research*, 1988, vol. 31, 241-246